# EUROPEAN PATENT SPECIFICATION

(11) **EP 3 249 042 B1**
(45) Date of publication and mention of the grant of the patent: **01.08.2018**
(21) Application number: 16889780.9
(22) Date of filing: 08.02.2016
(51) Int. Cl.: C12N 5/071

(54) **METHOD FOR PRODUCING MATURE SEBACEOUS GLAND CELLS**
VERFAHREN ZUR HERSTELLUNG VON REIFEN TALGDRÜSENZELLEN
PROCÉDÉ DE FABRICATION DE CELLULES DE GLANDE SÉBACÉE MATURES

(43) Date of publication of application: 29.11.2017
(73) Proprietor: Kao Corporation, Chuo-ku, Tokyo 103-8210 (JP)
(72) Inventor: INOUE, Takayoshi, Haga-gun, Tochigi 321-3497 (JP); HACHIYA, Akira, Haga-gun, Tochigi 321-3497 (JP)
(74) Representative: Vossius & Partner Patentanwälte Rechtsanwälte mbB
(86) International application number: PCT/JP2016/053692
(87) International publication number: WO 2017/138078

(56) References cited:
- JP-A- 2002 535 984
- JP-A- 2013 048 567
- JP-A- 2015 530 122
- SATO TAKASHI ET AL: "Adapalene suppresses sebum accumulationviathe inhibition of triacylglycerol biosynthesis and perilipin expression in differentiated hamster sebocytesin vitro", JOURNAL OF DERMATOLOGICAL SCIENCE, ELSEVIER, AMSTERDAM, NL, vol. 70, no. 3, 14 February 2013 (2013-02-14), pages 204-210, XP028543636, ISSN: 0923-1811, DOI: 10.1016/J.JDERMSCI.2013.02.003
- HONG I. ET AL.: 'LXRalpha enhances lipid synthesis in SZ95 sebocytes.' J INVEST DERMATOL. vol. 128, no. 5, 2007, pages 1266 - 72, XP055114192
- ROSENFIELD RL. ET AL.: 'Mechanisms of androgen induction of sebocyte differentiation' DERMATOLOGY vol. 196, no. 1, 1998, pages 43 - 6, XP009074036
- WANG B. ET AL.: 'Hypoxia induces leptin gene expression and secretion in human preadipocytes: differential effects of hypoxia on adipokine expression by preadipocytes' J ENDOCRINOL. vol. 198, no. 1, 07 May 2008, pages 127 - 34, XP055408987
- FINK T. ET AL.: 'Induction of adipocyte-like phenotype in human mesenchymal stem cells by hypoxia.' STEM CELLS vol. 22, no. 7, 2004, pages 1346 - 55, XP055408990
- REN H. ET AL.: 'Proliferation and differentiation of bone marrow stromal cells under hypoxic conditions' BIOCHEM BIOPHYS RES COMMUN vol. 347, no. 1, 18 August 2006, pages 12 - 21, XP024925463
- MORIZANE, M. ET AL.: 'Plin proteins play a key role in trophoblast lipid accumulation.' REPRODUCTIVE SCIENCES vol. 20, no. 3, March 2013, pages 113A - 114A, XP009501002

## Description

### Field of the Invention

The present invention relates to a method for producing differentiated sebaceous gland cells capable of forming lipid droplets.

### Background of the Invention

Sebaceous gland is a multicellular alveolar secretory portion, and it secretes sebum through sebaceous gland openings formed on the inner surfaces of skin pores. Sebaceous gland cells are present in the sebaceous gland and play a role for releasing sebum. The secreted sebum protects and moisturizes the skin and hair. Abnormal secretion of sebum causes skin troubles such as oily skin, dry skin, acne, seborrhea, seborrheic dermatitis, and xeroderma. In particular, acne is a skin trouble which many people in the world, especially, young people have experienced.

In order to conduct the research and development of pharmaceutical products or cosmetic products for controlling sebum secretion, it is important to construct a cultured cell model capable of reproducing the behavior of the sebaceous gland of a living animal. At present, as a sebaceous gland cell line which can be used in experiments, a hamster sebaceous gland cell line has been known. This cell line is advantageous in that it forms lipid droplets in cells, as with the sebaceous gland cells of a living body. On the other hand, since the genomic information of this cell line has not yet been elucidated, it is unsuitable for studies using a genetic approach.

Patent Literature 1 and Non Patent Literature 1 disclose a human-derived sebocyte cell line SZ95, which has been immortalized by transfection of SV-40 large T antigen DNA, and which is useful for the physiological inspection of the sebaceous gland. Since these cells are derived from humans, the genomic information thereof has been known, and it is advantageous as a study tool because it has high proliferation ability. On the other hand, the SZ95 cells are disadvantageous in that they hardly form lipid droplets therein.

In addition to the SZ95 cells disclosed in Patent Literature 1 and Non Patent Literature 1, all of the conventional human sebaceous gland cells have the following problem: the cells lose an ability to form lipid droplets if they are cultured *in vitro.* To date, it has been reported that the accumulation of lipids in cytoplasm is observed when sebaceous gland cells isolated from human skin are cultured, but that the amount of lipids accumulated is only about 4 to 8 times larger than in the case of keratinocytes, and thus that the amount is significantly lower than in the case of sebaceous gland cells *in vivo.* It is suggested because differentiation of sebaceous gland cells becomes incomplete in the case of *in-vitro* culture (Non Patent Literature 2). In accordance with this report, it has also been reported that if a Myc gene, which promotes differentiation of the above-mentioned SZ95 cells into sebaceous gland cells, is introduced into the SZ95 cells, lipid droplets are accumulated in the cytoplasm thereof (Non Patent Literature 3).

Patent Literature 2 describes that the cultured sebaceous gland cells comprise both differentiated cells which biosynthesize sebum, and undifferentiated cells which have proliferation ability but do not have ability to biosynthesize sebum; and this patent literature proposes an idea that the cultured sebaceous gland cells do not produce sebum because only undifferentiated cells proliferate as a result of subcultures and differentiated cells which biosynthesize sebum are thereby reduced. Patent Literature 2 also discloses a method including subjecting undifferentiated sebaceous gland cells to a gyratory culture, so as to induce differentiation of the cells.

Non Patent Literature 4 reports that when the human-derived sebocyte cell line SZ95 described in Non Patent Literature 1 was treated with linoleic acid, the accumulation of lipid droplets was observed in the cells. Non Patent Literature 5 discloses that the accumulation of lipid droplets was confirmed in the sebocyte cell line SEB-1 derived from normal skin from human preauricular area.

### Citation List

(Patent Literature 1) JP-A-2002-535984
(Patent Literature 2) JP-A-5-268948

(Non Patent Literature 1) J Invest Dermatol, 1999, 113: 1011-1120
(Non Patent Literature 2) Dermatology, 1998, 196: 21-31
(Non Patent Literature 3) Stem cell, 2008, 26: 1241-1252
(Non Patent Literature 4) J Invest Dermatol, 2008, 128: 1266-1272
(Non Patent Literature 5) J Invest Dermatol, 2003, 120: 905-914

### Summary of the Invention

The present invention provides a method for producing mature human sebaceous gland cells, comprising culturing immature human sebaceous gland cells under hypoxic conditions.

In addition, the present invention provides a method for inducing differentiation of immature human sebaceous gland cells into mature human sebaceous gland cells, comprising culturing the immature human sebaceous gland cells under hypoxic conditions.

Moreover, the present invention provides a method for evaluating and/or selecting a sebum secretion control agent, which comprises culturing immature human sebaceous gland cells under hypoxic conditions, and examining the influence of a test substance or test conditions on the formation of lipid droplets by the cultured cells.

Furthermore, the present disclosure provides a device for culturing mature human sebaceous gland cells, which comprises a chamber for culturing cells and an oxygen concentration controller for adjusting or maintaining the culture atmosphere to hypoxic conditions.

Further, the present disclosure provides a device for inducing differentiation of immature human sebaceous gland cells into mature human sebaceous gland cells, which comprises a chamber for culturing cells and an oxygen concentration controller for adjusting or maintaining the culture atmosphere to hypoxic conditions.

### Brief Description of the Drawings

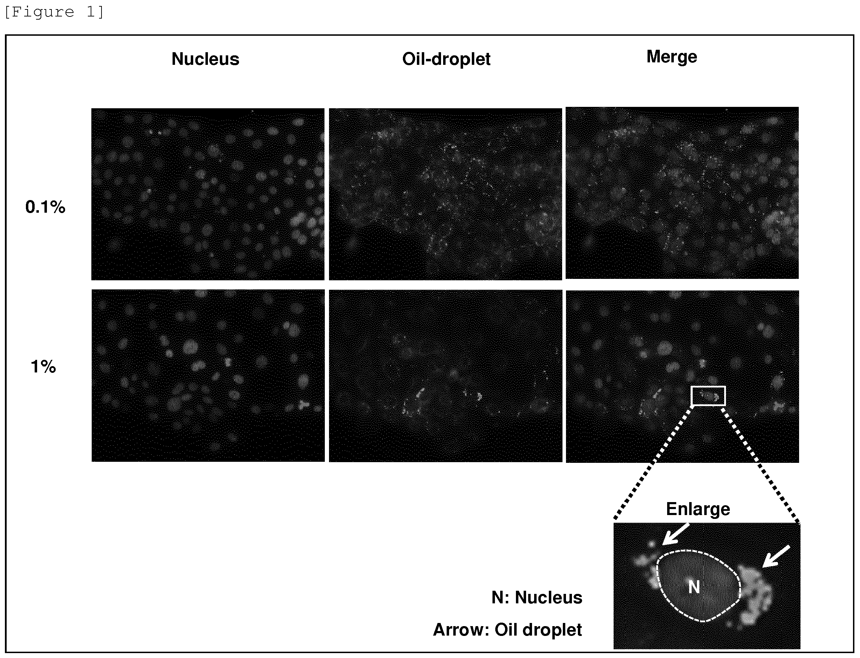

[Figure 1] Figure 1 shows the influence of the oxygen concentration on the formation of lipid droplets by sebaceous gland cells. Left: nucleus stained image, center: lipid droplet stained image, and right: merged image. The numerical value on the left side of the image indicates an oxygen concentration, and the term "Enlarged" indicates an enlarged view of a region surrounded with the white frame in the merged image showing a culture in an oxygen concentration of 1%.

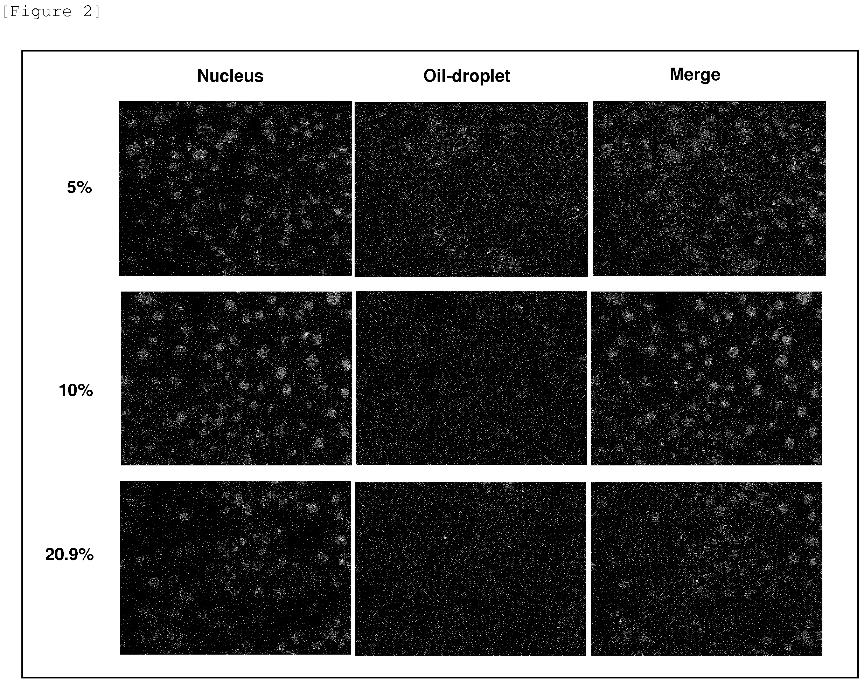

[Figure 2] Figure 2 shows the influence of the oxygen concentration on the formation of lipid droplets by sebaceous gland cells. Left: nucleus stained image, center: lipid droplet stained image, and right: merged image. The numerical value on the left side of the image indicates an oxygen concentration.

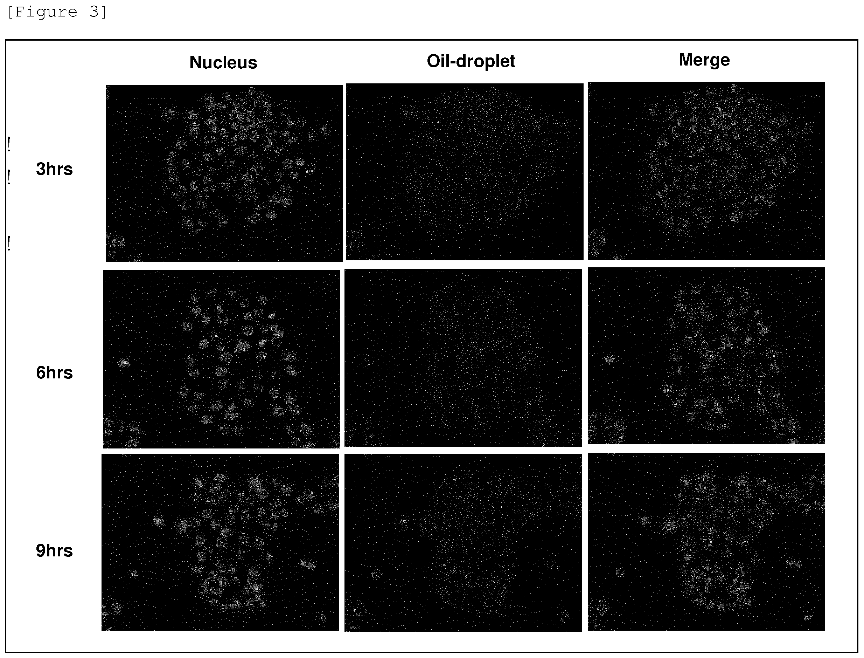

[Figure 3] Figure 3 shows a change in the amount of lipid droplets formed by sebaceous gland cells, which have been cultured under hypoxic conditions for a culture time of 3 to 9 hours. Left: nucleus stained image, center: lipid droplet stained image, and right: merged image.

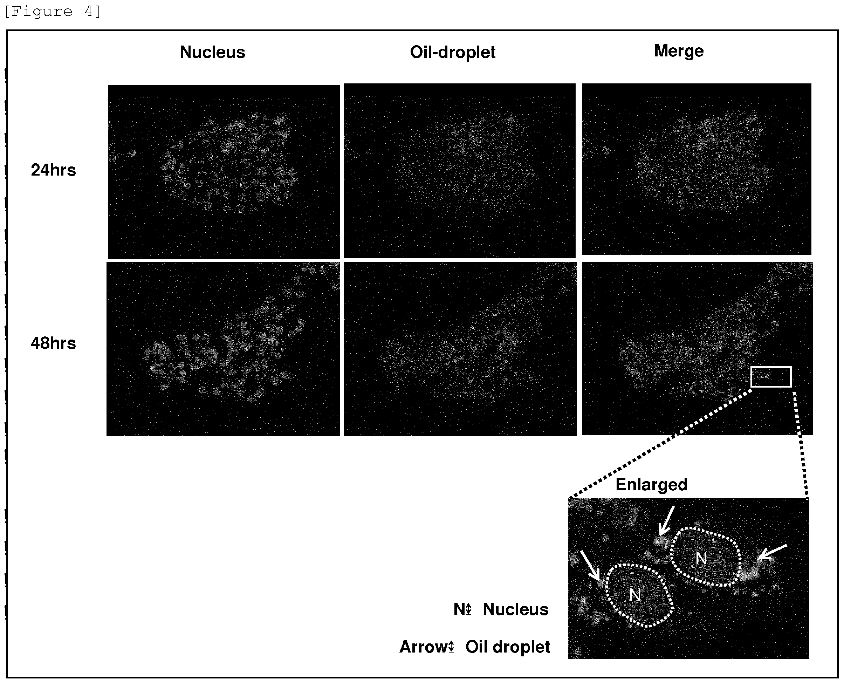

[Figure 4] Figure 4 shows a change in the amount of lipid droplets formed by sebaceous gland cells, which have been cultured under hypoxic conditions for a culture time of 24 to 48 hours. Left: nucleus stained image, center: lipid droplet stained image, and right: merged image. The term "Enlarged" indicates an enlarged view of a region surrounded with the white frame in the merged image showing a culture for 48 hours.

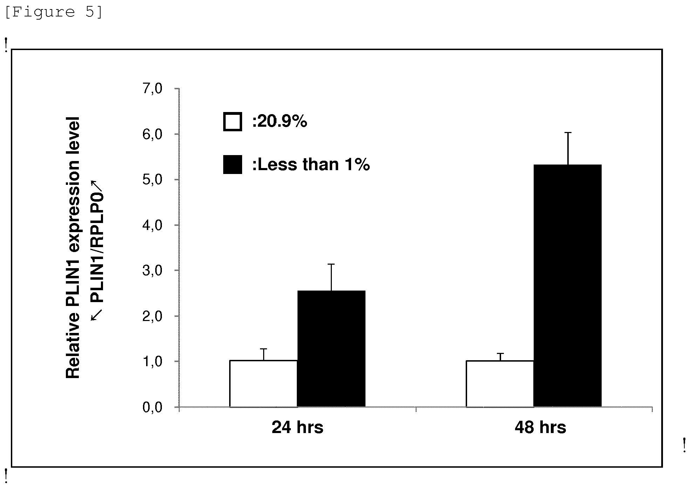

[Figure 5] Figure 5 shows the improvement of the expression level of PLIN1 in sebaceous gland cells, which have been cultured under hypoxic conditions. Error bar = SD, and n = 3.

### Detailed Description of the Invention

The present invention relates to a method for producing differentiated, cultured sebaceous gland cells, which are capable of forming lipid droplets.

The present inventors have conducted intensive studies regarding a method for imparting an ability to form lipid droplets, as in the case of sebaceous gland cells *in vivo,* to conventional cultured human sebaceous gland cells. As a result, the inventors found that human sebaceous gland cells differentiate into cells forming lipid droplets by culturing the human sebaceous gland cells under hypoxic conditions.

According to the present invention, cultured mature human sebaceous gland cells, which have acquired the property of forming lipid droplets, as with sebaceous gland cells *in vivo,* can be provided. The cultured mature human sebaceous gland cells, which are provided by the present invention, can become useful tools for studies regarding the mechanism of sebum secretion or the development of pharmaceutical products or cosmetic products for controlling sebum secretion.

In the outermost layer of the sebaceous gland, basal cells, which are undifferentiated sebaceous gland cells, are present and vigorously carry out cell division. After the basal cells have moved to the central portion of the sebaceous gland, the cell division is gradually terminated. Cells, which have terminated cell division, start to accumulate lipid droplets therein, and eventually, a major part of cytoplasm is filled with lipid droplets. The sebaceous gland cells, which comprise such lipid droplets, are differentiated sebaceous gland cells (J. Invest. Dermatol, 1974, 62: 147-152). The differentiated sebaceous gland cells comprising lipid droplets are finally disintegrated and as a result, release sebum.

In the present description, the term "mature sebaceous gland cells" is used to mean differentiated sebaceous gland cells, which have lipid droplets therein. In addition, in the present description, the term "immature sebaceous gland cells" is used to distinguish the immature sebaceous gland cells from the above described "mature sebaceous gland cells," and this term means undifferentiated sebaceous gland cells, which are not the above described "mature sebaceous gland cells," or sebaceous gland cells, which do not have lipid droplets therein.

The presence of lipid droplets in a cell can be confirmed by a known method. For example, as described in the after-mentioned examples, the presence of such lipid droplets can be confirmed by subjecting cells, which have been stained with a fat staining reagent such as Oil Red O or Nile Red, to microscopic observation, or by performing a fluorescence or absorbance measurement. For instance, when lipid droplets are present in a cell, a clear dot-shaped or circular stained region is observed in the cytoplasm of the cell, which has been treated with a fat staining reagent (for example, Figure 1). Otherwise, the presence of lipid droplets in a cell can also be confirmed by, for example, measuring the expression level of a lipid droplet-associated protein, such as perilipin 1 (PLIN1), in the cell (PNAS, 2001, 98: 6494-6499).

In the present invention, differentiation of immature human sebaceous gland cells, in which lipid droplets are neither formed nor accumulated, is induced, and as a result, mature human sebaceous gland cells, in which lipid droplets are formed or accumulated, or the amount of lipid droplets accumulated is increased, are prepared. That is to say, in one aspect, the present invention relates to a method for producing mature human sebaceous gland cells. In another aspect, the present invention relates to a method for inducing differentiation of immature human sebaceous gland cells into mature human sebaceous gland cells. These methods of the present invention comprise culturing immature human sebaceous gland cells under hypoxic conditions.

In the above described methods of the present invention, examples of the immature human sebaceous gland cells, which are to be subjected to a culture under hypoxic conditions, include: cultured human sebaceous gland cells prepared from the skin of a living body comprising a sebaceous gland according to a known method; and an established human sebaceous gland cell line. The immature human sebaceous gland cells are preferably an established human sebaceous gland cell line. Examples of the human sebaceous gland cell line include, but are not limited to, immortalized human-derived sebocyte cell lines as described in Patent Literature 1 or Non Patent Literatures 1 and 5 (e.g., DSM ACC2383, SZ95, and SEB-1).

The above described cultured human sebaceous gland cells and cell lines may include not only immature sebaceous gland cells, but also some mature sebaceous gland cells. However, if a mixture of such immature cells and mature cells is cultured according to the method of the present invention, the cultured sebaceous gland cells, in which the content percentage of mature human sebaceous gland cells comprising lipid droplets has been significantly increased, can be prepared. Accordingly, the immature sebaceous gland cells cultured by the method of the present invention may be either a sebaceous gland cell population consisting of only immature sebaceous gland cells, or a sebaceous gland cell population comprising immature sebaceous gland cells. In other words, in one embodiment, the method for producing mature human sebaceous gland cells of the present invention may be a method for producing mature human sebaceous gland cells from immature human sebaceous gland cells; and in another embodiment, the method for producing mature human sebaceous gland cells of the present invention may be a method for producing a human sebaceous gland cell culture having a higher content percentage of mature human sebaceous gland cells, from a human sebaceous gland cell culture comprising immature human sebaceous gland cells.

In the above described method of the present invention, the above described immature human sebaceous gland cells are cultured under hypoxic conditions. Thereby, the immature cells are induced to differentiate into mature human sebaceous gland cells. The hypoxic conditions in the present invention are conditions under which the oxygen concentration in the culture atmosphere is preferably approximately 5% or less, more preferably approximately 2% or less, and further preferably approximately 1% or less, or conditions under which the oxygen concentration in the culture atmosphere is from approximately 0.1% to approximately 5%. Such hypoxic conditions can be achieved using an incubator equipped with an oxygen concentration controller. Such an incubator is known, and various types of incubators are commercially available (for example, BIONIX hypoxic culture kit; SUGIYAMA-GEN CO., LTD.).

With regard to other conditions applied to the culture of human sebaceous gland cells according to the above described method of the present invention, conditions for the *in-vitro* culture of ordinary human sebaceous gland cells may be applied. Examples of the culture conditions will be given below: As a medium, a basal medium, such as William's E Medium, D-MEM, RPMI1640, Ham's F-12, or Modified D-MEM/Ham's F-12 (1 : 1) medium (e.g., Sebomed™ basal medium), is used; and preferably, Modified D-MEM/Ham's F-12 (1 : 1) medium, to which from 0.1% to 20% of, preferably 10% of serum, from 1 to 10 ng/mL of, preferably from 3 to 6 ng/mL of Epidermal Growth Factor (EGF), from 0.1 to 20 µg/mL of, preferably from 5 to 15 µg/mL of hydrocortisone, and from 5 to 50 µg/mL of, preferably from 8 to 20 µg/mL of insulin have been added, is used. As a culture vessel, a plastic, a glass or the like, and preferably, a plastic is used. The concentration of cells seeded on a medium is from 1 × 10³ to 1 × 10⁶ cells/cm², and preferably from 0.5 × 10³ cells/cm² to 0.5 × 10⁵ cells/cm². The culture is carried out at a culture temperature of from 30°C to 40°C, and preferably from 33°C to 38°C, under static, shaking or gyratory culture conditions, and preferably in a static state. The culture time may be determined, as appropriate, depending on the state of lipid droplets formed in cells, and it is preferably 24 hours or more, and more preferably approximately from 24 to 72 hours.

Accordingly, in another aspect, the present invention relates to the use of a cell culture device comprising a means for culturing cells under the above described hypoxic conditions, for use in the culture of mature human sebaceous gland cells, or induction of immature human sebaceous gland cells to differentiate into mature human sebaceous gland cells. In one embodiment, the cell culture device comprises a chamber for culturing cells and an oxygen concentration controller for adjusting or maintaining the culture atmosphere to hypoxic conditions.

The chamber used for the above described cell culture is not particularly limited, as long as it is capable of blocking the atmosphere in which cells are cultured, from the normal ambient atmosphere. Examples of the chamber include a hermetically sealed petri dish, jar, and incubator. The above described oxygen concentration controller is a device capable of adjusting or maintaining the oxygen concentration in the above described chamber to a concentration lower than the normal atmosphere, and preferably to the aforementioned hypoxic conditions. Examples of the oxygen concentration controller include an oxygen absorber, and an oxygen controller capable of mixing oxygen, nitrogen and carbon dioxide at any given concentrations. The above-exemplified chamber and oxygen concentration controller are commercially available, and further, a hypoxic culture kit, in which the two above devices are combined, is also commercially available (e.g., BIONIX hypoxic culture kit; SUGIYAMA-GEN CO., LTD.). For example, apparatuses or kits for hypoxic culture, which have been conventionally provided for use in the culture of cancer cells, can be applied as the device for culturing mature human sebaceous gland cells of the present invention.

Since the mature human sebaceous gland cells obtained according to the aforementioned procedures have the property of forming or accumulating lipid droplets as with sebaceous gland cells *in vivo,* these cells can be used as human sebaceous gland models in studies regarding the mechanism of sebum secretion, or in the research and development of pharmaceutical products or cosmetic products for controlling sebum secretion.

Therefore, in another aspect, the present invention relates to a method for evaluating and/or selecting a sebum secretion control agent by utilizing the above described human sebaceous gland cells cultured under hypoxic conditions, which have the property of forming or accumulating lipid droplets. This method comprises culturing immature human sebaceous gland cells under hypoxic conditions, and examining the influence of a test substance or test conditions on the formation of lipid droplets by the cultured cells. The procedures for culturing the immature human sebaceous gland cells under hypoxic conditions are as described above.

In one embodiment of the method for evaluating and/or selecting a sebum secretion control agent of the present invention, the above described culture of immature human sebaceous gland cells under hypoxic conditions is carried out in the presence of a test substance. After completion of the culture for a predetermined period of time, lipid droplets in the cultured cells are observed, and the influence of the test substance on the formation of lipid droplets by the cells is then examined.

In another embodiment, the above described culture of immature human sebaceous gland cells under hypoxic conditions is carried out under the application of test conditions. After completion of the culture for a predetermined period of time, lipid droplets in the cultured cells are observed, and the influence of the test conditions on the formation of lipid droplets by the cells is then examined.

In another embodiment, the above described culture of immature human sebaceous gland cells under hypoxic conditions is carried out without the application of a test substance or test conditions, and thereafter, the cultured cells are further cultured under the application of a test substance or test conditions. This culture after the application of a test substance or test conditions is not necessarily carried out under hypoxic conditions. After completion of the culture for a predetermined period of time, lipid droplets in the cultured cells are observed, and the influence of the test substance or the test conditions on the formation of lipid droplets by the cells is then examined.

The test substance or the test conditions are not particularly limited, as long as they are a substance or conditions which are desirably used for the control of sebum secretion. The test substance may be a naturally occurring substance, or may also be a substance artificially synthesized by a chemical or biological method, etc. Otherwise, the test substance may also be a compound, a composition, or a mixture. Examples of the test conditions include physicochemical conditions such as light (e.g., light irradiation or light shielding) or temperature (e.g., heating or cooling), but are not limited thereto.

Examples of the method of applying a test substance or test conditions to the cultured cells include a method of adding a test substance to a medium, a method of directly adding test substance to cells, a method of culturing cells in an atmosphere in which a test substance is present, and a method of exposing cells during culture to predetermined physiochemical conditions, but are not limited thereto.

Upon observation of lipid droplets in cells, such lipid droplets may be qualitatively evaluated by microscopic observation. Alternatively, the amount of lipid droplets formed by cells may also be quantified. Examples of the method of quantifying the amount of lipid droplets formed by cells include a method of quantifying the oil and fat amount, based on the staining intensity of cells stained with a fat staining reagent such as Oil Red O or Nile Red, which has been measured using a fluorescence or absorbance measurement device, and a method of quantifying the expression level of a lipid droplet-associated protein, such as perilipin 1 (PLIN1), in cells, according to real-time RT-PCR or the like.

Subsequently, the influence of the test substance or the test conditions on the formation of lipid droplets by the cells is examined. When the amount of lipid droplets formed by the cultured cells has been increased or decreased by application of a test substance or test conditions, the test substance or the test conditions are selected as a sebum secretion control agent. More specifically, when the amount of lipid droplets formed by the cultured cells has been increased by application of a test substance or test conditions, the test substance or the test conditions are selected as a sebum secretion activator. On the other hand, when the amount of lipid droplets formed by the cultured cells has been decreased by application of a test substance or test conditions, the test substance or the test conditions are selected as a sebum secretion inhibitor.

Preferably, the amount of lipid droplets formed by cells is compared with a control. When the amount of lipid droplets formed by cells, which had been cultured under the application of a test substance or test conditions, has been statistically significantly increased in comparison with the case of control cells, the test substance or the test conditions are selected as a sebum secretion activator. On the other hand, when the amount of lipid droplets formed by cells, which had been cultured under the application of a test substance or test conditions, has been statistically significantly decreased in comparison with the case of control cells, the test substance or the test conditions are selected as a sebum secretion inhibitor. Otherwise, when the amount of lipid droplets formed by cells, which had been cultured under the application of a test substance or test conditions, has been increased to preferably 120% or more, and more preferably 150% or more, in comparison to the case of control cells, the test substance or the test conditions are selected as a sebum secretion activator. On the other hand, when the amount of lipid droplets formed by cells, which had been cultured under the application of a test substance or test conditions, has been decreased to preferably 80% or less, and more preferably 60% or less, in comparison to the case of control cells, the test substance or the test conditions are selected as a sebum secretion inhibitor.

Examples of such a control include: immature human sebaceous gland cells cultured under hypoxic conditions without the application of a test substance or test conditions, or in the presence of a control substance; and cells, to which a test substance or test conditions have not been applied during or after the culture of the cells under hypoxic conditions.

The selected sebum secretion control agent has the effect of activating or inhibiting sebum secretion, and is able to treat or ameliorate various conditions or diseases associated with excessive sebum secretion or a reduction in the sebum secretion. For instance, the sebum secretion inhibitor can be used as an agent for treating or ameliorating acne, oily skin, seborrhea and the like, or as an active ingredient for suppressing odor (body odor, etc.) caused by sebum, whereas the sebum secretion activator can be used as an active ingredient of an agent for treating or ameliorating dry skin, xeroderma, dry eyes, roughened lips, dry lips, etc.

The present invention also includes, as illustrative embodiments, the following substances, production methods, intended uses, or methods. However, these embodiments are not intended to limit the scope of the present invention.
<1> A method for producing mature human sebaceous gland cells, comprising culturing immature human sebaceous gland cells under hypoxic conditions.
<2> A method for inducing differentiation of immature human sebaceous gland cells into mature human sebaceous gland cells, comprising culturing the immature human sebaceous gland cells under hypoxic conditions.
<3> A method for increasing the amount of lipid droplets formed by human sebaceous gland cells, comprising culturing immature human sebaceous gland cells under hypoxic conditions.
<4> The method according to any one of the above <1> to <3>, wherein preferably, the above described immature human sebaceous gland cells are a sebaceous gland cell population consisting of only immature sebaceous gland cells, or a sebaceous gland cell population comprising immature sebaceous gland cells.
<5> The method according to any one of the above <1> to <3>, wherein the above described immature human sebaceous gland cells are preferably a human sebaceous gland cell line, and more preferably cells registered under Accession No. DSM ACC2383, SZ95 cells, or SEB-1 cells.
<6> The method according to any one of the above <1> to <5>, wherein the above described hypoxic conditions are conditions in which the oxygen concentration in the culture atmosphere is preferably approximately 5% or less, more preferably approximately 2% or less, and further preferably approximately 1% or less, or conditions in which the oxygen concentration in the culture atmosphere is from approximately 0.1% to approximately 5%.
<7> The method according to any one of the above <1> to <6>, wherein preferably, the above described culture is carried out in a static state.
<8> The method according to any one of the above <1> to <7>, wherein preferably, the above described culture is carried out for 24 hours or more.
<9> A method for evaluating and/or selecting a sebum secretion control agent, which comprises culturing immature human sebaceous gland cells under hypoxic conditions, and examining the influence of a test substance or test conditions on the formation of lipid droplets by the cultured cells.
<10> The method according to the above <9>, wherein preferably, the above described immature human sebaceous gland cells are a sebaceous gland cell population consisting of only immature sebaceous gland cells, or a sebaceous gland cell population comprising immature sebaceous gland cells.
<11> The method according to the above <9>, wherein the above described immature human sebaceous gland cells are preferably a human sebaceous gland cell line, and more preferably cells registered under Accession No. DSM ACC2383, SZ95 cells, or SEB-1 cells.
<12> The method according to any one of the above <9> to <11>, wherein the above described hypoxic conditions are conditions in which the oxygen concentration in the culture atmosphere is preferably approximately 5% or less, more preferably approximately 2% or less, and further preferably approximately 1% or less, or conditions in which the oxygen concentration in the culture atmosphere is from approximately 0.1% to approximately 5%.
<13> The method according to any one of the above <9> to <12>, wherein preferably the above described culture is carried out in a static state.
<14> The method according to any one of the above <9> to <13>, wherein preferably, the above described culture is carried out for 24 hours or more.
<15> The method according to any one of the above <9> to <14>, wherein preferably, the above described culture of immature human sebaceous gland cells under hypoxic conditions is carried out in the presence of a test substance or under the application of test conditions.
<16> The method according to any one of the above <9> to <14>, wherein preferably, the above described culture of immature human sebaceous gland cells under hypoxic conditions is carried out without the application of a test substance or test conditions, and the above described method further comprises further culturing the cells, which have been cultured under the hypoxic conditions, under the application of a test substance or test conditions.
<17> The method according to any one of the above <9> to <16>, which preferably further comprises selecting a test substance or test conditions as a sebum secretion control agent, when the amount of lipid droplets formed by the cultured cells has been increased or decreased by the application of the test substance or the test conditions.
<18> The method according to the above <15>, which preferably further comprises:
   culturing immature human sebaceous gland cells under hypoxic conditions, without the application of a test substance or test conditions, and
   any one of the following steps:
      selecting a test substance or test conditions as a sebum secretion activator, when the amount of lipid droplets formed by cells, which had been cultured under the application of the above described test substance or test conditions, has been statistically significantly increased in comparison to that formed by cells, which had been cultured without the application of the test substance or the test conditions;
      selecting a test substance or test conditions as a sebum secretion inhibitor, when the amount of lipid droplets formed by cells, which had been cultured under the application of the above described test substance or test conditions, has been statistically significantly decreased in comparison to that formed by cells, which had been cultured without the application of the test substance or the test conditions;
      selecting a test substance or test conditions as a sebum secretion activator, when the amount of lipid droplets formed by cells, which had been cultured under the application of the above described test substance or test conditions, has been increased to preferably 120% or more, and more preferably 150% or more, in comparison to that formed by cells, which had been cultured without the application of the test substance or the test conditions; and
      selecting a test substance or test conditions as a sebum secretion inhibitor, when the amount of lipid droplets formed by cells, which had been cultured under the application of the above described test substance or test conditions, has been decreased to preferably 80% or less, and more preferably 60% or less, in comparison to that formed by cells, which had been cultured without the application of the test substance or the test conditions.
<19> The method according to the above <16>, which preferably further comprises:
   further culturing the above described cells, which had been cultured under the hypoxic conditions, without the application of a test substance or test conditions, and
   any one of the following steps:
      selecting a test substance or test conditions as a sebum secretion activator, when the amount of lipid droplets formed by cells, which had been cultured under the application of the above described test substance or test conditions, has been statistically significantly increased in comparison to that formed by cells, which had been cultured without the application of the test substance or the test conditions;
      selecting a test substance or test conditions as a sebum secretion inhibitor, when the amount of lipid droplets formed by cells, which had been cultured under the application of the above described test substance or test conditions, has been statistically significantly decreased in comparison to that formed by cells, which had been cultured without the application of the test substance or the test conditions;
      selecting a test substance or test conditions as a sebum secretion activator, when the amount of lipid droplets formed by cells, which had been cultured under the application of the above described test substance or test conditions, has been increased to preferably 120% or more, and more preferably 150% or more, in comparison to that formed by cells, which had been cultured without the application of the test substance or the test conditions; and
      selecting a test substance or test conditions as a sebum secretion inhibitor, when the amount of lipid droplets formed by cells, which had been cultured under the application of the above described test substance or test conditions, has been decreased to preferably 80% or less, and more preferably 60% or less, in comparison to that formed by cells, which had been cultured without the application of the test substance or the test conditions.
<20> The method according to any one of the above <9> to <19>, wherein preferably, the above described sebum secretion control agent is an agent for treating or ameliorating various conditions or diseases, which are associated with excessive sebum secretion or a reduction in the sebum secretion.
<21> A device for culturing mature human sebaceous gland cells, which comprises a chamber for culturing cells and an oxygen concentration controller for adjusting or maintaining the culture atmosphere to hypoxic conditions.
<22> A device for inducing differentiation of immature human sebaceous gland cells into mature human sebaceous gland cells, which comprises a chamber for culturing cells and an oxygen concentration controller for adjusting or maintaining the culture atmosphere to hypoxic conditions.
<23> The device according to the above <21> or <22>, wherein the above described hypoxic conditions are conditions in which the oxygen concentration in the culture atmosphere is preferably approximately 5% or less, more preferably approximately 2% or less, and further preferably approximately 1% or less, or conditions under which the oxygen concentration in the culture atmosphere is from approximately 0.1% to approximately 5%.

### Examples

Hereinafter, the present invention will be described more in detail based on the following examples. However, these examples are not intended to limit the scope of the present invention.

### <Method>

### 1) Cells

A human sebaceous gland cell line (SZ95) was cultured in Sebomed™ basal medium containing 10% FBS and 5 ng/mL EGF. The culture under the normal oxygen concentration was carried out in an oxygen concentration (20.9%) in the atmosphere. The culture under hypoxic conditions was carried out using BIONIX hypoxic culture kit (SUGIYAMA-GEN CO., LTD.) under conditions of an oxygen concentration of 10%, 5%, 1% or 0.1%. In all cases, the cells were cultured in 5% CO₂ at a temperature of 37°C for at maximum 72 hours.

### 2) Fluorescence microscopic observation of lipid droplets

The SZ95 cells were immobilized with a 4% paraformaldehyde solution for 5 minutes, and were then stained with a 1 µg/mL Nile red solution (Sigma Aldrich) and a DAPI solution (Life Technologies) for 15 minutes at a room temperature. Thereafter, lipid droplets were observed under a fluorescence microscope.

### 3) Real-time RT-PCR

Total RNA was extracted from the cells using RNeasy (registered trademark) Mini kit, in accordance with protocols included with the kit. For the synthesis of cDNA from the extracted total RNA, QuantiTect (registered trademark) reverse transcription kit (QIAGEN) was used. In real-time PCR, TaqMan (registered trademark) Universal master Mix (Life technologies) and TaqMan probe (Life technologies) were used, and the expression level of mRNA of Perilipin 1 (PLIN1) and of Ribosomal protein LP0 (RPLP0) was quantified. The expression level of mRNA of PLIN1 was corrected based on the expression level of RPLP0.

### (Test 1) Activation of the formation of lipid droplets by sebaceous gland cells in the culture of the cells under hypoxic conditions

SZ95 cells were cultured for 2 days in an oxygen concentration of each of 20.9%, 10%, 5%, 1% and 0.1%, and were then stained with Nile red, followed by performing observation under a fluorescence microscope. The obtained microscopic images are shown in Figures 1 and 2. In Figures 1 and 2, the circular region with low brightness (the region enclosed with the dotted line in the enlarged view of Figure 1), which is shown as white, indicates the nucleus of a cell, and the white dots with high brightness (indicates with the arrows in the enlarged view of Figure 1), which are present in large quantities around the nucleus, indicate lipid droplets. As a result of the microscopic observation, the cells, which were cultured in oxygen concentrations of 0.1%, 1% and 5%, were strongly stained with Nile red, and thus it became clear that lipid droplets were accumulated in the cells. Among others, it was confirmed that the cells cultured in the lowest oxygen concentration (0.1%) had the highest lipid droplet formation percentage.

### (Test 2) Change in the amount of lipid droplets formed in sebaceous gland cells, depending on culture time

SZ95 cells were cultured in an oxygen concentration of 0.1% for 3, 6, 9, 24 and 48 hours. As a result, the cells cultured for 24 hours or more were strongly stained with Nile red, and thus, it became clear that lipid droplets were abundantly accumulated in the cells (Figures 3 and 4).

### (Test 3) Molecular mechanism of lipid droplet formation under hypoxic conditions

It had been reported that the amount of lipid droplets, which are generally accumulated in adipocytes, are significantly decreased in Perilipin 1 (PLIN1) knockout mice (PNAS, 2001, 98: 6494-6499). In the present test, SZ95 cells were cultured under conditions of an oxygen concentration of 20.9% (normal) and 0.1% (low oxygen) for 24 or 48 hours, and thereafter, the expression level of PLIN1 in the cells was measured. As a result, it became clear that the expression of PLIN1 was significantly increased in the cells cultured under hypoxic conditions (0.1%), in comparison to under normal oxygen concentration (20.9%) conditions (Figure 5). These results suggested that PLIN1 is associated with the molecular mechanism of lipid droplet formation in sebaceous gland cells under a hypoxic environment, and that the amount of lipid droplets formed in sebaceous gland cells can be quantified using PLIN1 as an indicator.

## Claims

1. A method for producing mature human sebaceous gland cells, or for inducing differentiation of immature human sebaceous gland cells into mature human sebaceous gland cells, the method comprising culturing immature human sebaceous gland cells under hypoxic conditions.

2. A method for evaluating and/or selecting a sebum secretion control agent, which comprises culturing immature human sebaceous gland cells under hypoxic conditions, and examining the influence of a test substance or test conditions on the accumulation of lipid droplets in the cultured cells.

3. The method according to claim 1 or 2, wherein the immature human sebaceous gland cells are a human sebaceous gland cell line.

4. The method according to claim 3, wherein the human sebaceous gland cell line is cells registered under Accession No. DSM ACC2383, SZ95 cells, or SEB-1 cells.

5. The method according to any one of claims 1 to 4, wherein the hypoxic conditions are conditions in which the oxygen concentration in the culture atmosphere is 5% or less.

6. The method according to any one of claims 1 to 4, wherein the hypoxic conditions are conditions in which the oxygen concentration in the culture atmosphere is from 0.1% to 5%.

7. The method according to any one of claims 1 to 6, wherein the culture is carried out in a static state.

8. The method according to any one of claims 1 to 7, wherein the culture is carried out for 24 hours or more.

9. The method according to claim 2, wherein the culture of immature human sebaceous gland cells under hypoxic conditions is carried out under the application of the test substance or the test conditions.

10. The method according to claim 9, wherein the method further comprises selecting a test substance or test conditions as a sebum secretion control agent when the amount of lipid droplets formed by the cultured cells has been increased or decreased by the application of the test substance or the test conditions.

11. The method according to claim 2, wherein the above described culture of immature human sebaceous gland cells under hypoxic conditions is carried out without the application of a test substance or test conditions, and the method further comprises further culturing the cells, which have been cultured under the hypoxic conditions, under the application of a test substance or test conditions.

12. The method according to claim 11, wherein the method further comprises selecting a test substance or test conditions as a sebum secretion control agent, when the amount of lipid droplets formed by the cultured cells has been increased or decreased by the application of the test substance or the test conditions.

## Patentansprüche

1. Verfahren zum Herstellen reifer humaner Talgdrüsenzellen, oder zur Induzierung der Differenzierung unreifer humaner Talgdrüsenzellen in reife humane Talgdrüsenzellen, wobei das Verfahren umfasst: Züchten unreifer humaner Talgdrüsenzellen unter hypoxischen Bedingungen.

2. Verfahren zum Bewerten und/oder Auswählen eines Kontrollagens der Talgsekretion, das umfasst: Züchten unreifer humaner Talgdrüsenzellen unter hypoxischen Bedingungen, und Untersuchen des Einflusses einer Testsubstanz oder von Testbedingungen auf die Anhäufung von Lipidtröpfchen in den gezüchteten Zellen.

3. Verfahren nach Anspruch 1 oder 2, wobei die unreifen humanen Talgdrüsenzellen eine humane Talgdrüsenzelllinie sind.

4. Verfahren nach Anspruch 3, wobei die humane Talgdrüsenzelllinie unter der Hinterlegungsnummer DSM ACC2383 registrierte Zellen, SZ95-Zellen, oder SEB-1-Zellen sind.

5. Verfahren nach einem der Ansprüche 1 bis 4, wobei die hypoxischen Bedingungen Bedingungen sind, unter denen die Sauerstoffkonzentration in der Kulturatmosphäre 5% oder weniger ist.

6. Verfahren nach einem der Ansprüche 1 bis 4, wobei die hypoxischen Bedingungen Bedingungen sind, unter denen die Sauerstoffkonzentration in der Kulturatmosphäre von 0.1% bis 5% ist.

7. Verfahren nach einem der Ansprüche 1 bis 6, wobei das Züchten in einem statischen Zustand durchgeführt wird.

8. Verfahren nach einem der Ansprüche 1 bis 7, wobei das Züchten für 24 Stunden oder länger durchgeführt wird.

9. Verfahren nach Anspruch 2, wobei das Züchten der unreifen humanen Talgdrüsenzellen unter hypoxischen Bedingungen unter Verwendung einer Testsubstanz oder von Testbedingungen durchgeführt wird.

10. Verfahren nach Anspruch 9, wobei das Verfahren des Weiteren umfasst: Auswählen einer Testsubstanz oder von Testbedingungen als ein Kontrollagens der Talgsekretion, wenn die Menge an durch die gezüchteten Zellen gebildeter Lipidtröpfchen durch die Verwendung der Testsubstanz oder der Testbedingungen erhöht oder verringert worden ist.

11. Verfahren nach Anspruch 2, wobei das oben beschriebene Züchten unreifer humaner Talgdrüsenzellen unter hypoxischen Bedingungen ohne Verwendung einer Testsubstanz oder von Testbedingungen durchgeführt wird und das Verfahren des Weiteren umfasst: weiteres Züchten der Zellen, die unter den hypoxischen Bedingungen gezüchtet worden sind, unter Verwendung einer Testsubstanz oder von Testbedingungen.

12. Verfahren nach Anspruch 11, wobei das Verfahren des Weiteren umfasst: Auswählen einer Testsubstanz oder von Testbedingungen als ein Kontrollagens der Talgsekretion, wenn die Menge an durch die gezüchteten Zellen gebildeter Lipidtröpfchen durch Verwendung der Testsubstanz oder der Testbedingungen erhöht oder verringert worden ist.

## Revendications

1. Méthode pour produire des cellules matures de glandes sébacées humaines, ou pour induire la différenciation de cellules immatures de glandes sébacées humaines en cellules matures de glandes sébacées humaines, la méthode comprenant la culture de cellules immatures de glandes sébacées humaines dans des conditions hypoxiques.

2. Méthode pour évaluer et/ou sélectionner un agent de contrôle de la sécrétion de sébum, qui comprend la culture de cellules immatures de glandes sébacées humaines dans des conditions hypoxiques, et l'examen de l'influence d'une substance à tester ou de conditions à tester sur l'accumulation de gouttelettes lipidiques dans les cellules cultivées.

3. Méthode selon la revendication 1 ou 2, dans laquelle les cellules immatures de glandes sébacées humaines sont une lignée cellulaire de glandes sébacées humaines.

4. Méthode selon la revendication 3, dans laquelle la lignée cellulaire de glandes sébacées humaines consiste en des cellules enregistrées sous le No. d'accession DSM ACC2383, des cellules SZ95, ou des cellules SEB-1.

5. Méthode selon l'une quelconque des revendications 1 à 4, dans laquelle les conditions hypoxiques sont des conditions dans lesquelles la concentration en oxygène dans l'atmosphère de culture est de 5 % ou moins.

6. Méthode selon l'une quelconque des revendications 1 à 4, dans laquelle les conditions hypoxiques sont des conditions dans lesquelles la concentration en oxygène dans l'atmosphère de culture est de 0,1 % à 5 %.

7. Méthode selon l'une quelconque des revendications 1 à 6, dans laquelle la culture est réalisée dans un état statique.

8. Méthode selon l'une quelconque des revendications 1 à 7, dans laquelle la culture est réalisée pendant 24 heures ou plus.

9. Méthode selon la revendication 2, dans laquelle la culture des cellules immatures de glandes sébacées humaines dans des conditions hypoxiques est réalisée sous l'application de la substance à tester ou des conditions à tester.

10. Méthode selon la revendication 9, dans laquelle la méthode comprend en outre la sélection d'une substance à tester ou de conditions à tester en tant qu'agent de contrôle de la sécrétion de sébum lorsque la quantité des gouttelettes lipidiques formées par les cellules cultivées a été augmentée ou diminuée par l'application de la substance à tester ou des conditions à tester.

11. Méthode selon la revendication 2, dans laquelle la culture décrite ci-dessus de cellules immatures de glandes sébacées humaines dans des conditions hypoxiques est réalisée sans l'application d'une substance à tester ou de conditions à tester, et la méthode comprend en outre la culture supplémentaire des cellules, qui ont été cultivées dans les conditions hypoxiques, sous l'application d'une substance à tester ou de conditions à tester.

12. Méthode selon la revendication 11, dans laquelle la méthode comprend en outre la sélection d'une substance à tester ou de conditions à tester en tant qu'agent de contrôle de la sécrétion de sébum, lorsque la quantité des gouttelettes lipidiques formées par les cellules cultivées a été augmentée ou diminuée par l'application de la substance à tester ou des conditions à tester.
